Europäisches Patentamt

European Patent Office

Office européen des brevets

(19)

(11) Veröffentlichungsnummer: **0 175 247**
**B1**

(12) # EUROPÄISCHE PATENTSCHRIFT

(45) Veröffentlichungstag der Patentschrift:
**07.12.88**

(21) Anmeldenummer: **85111341.5**

(22) Anmeldetag: **07.09.85**

(51) Int. Cl.⁴: **C 07 D 307/89,** C 07 D 307/60 //
F04F5/02

(54) **Verfahren zur Erzeugung von Unterdruck in Apparaten bei der Herstellung von Phthalsäureanhydrid und Maleinsäureanhydrid.**

(30) Priorität: **12.09.84 DE 3433401**

(43) Veröffentlichungstag der Anmeldung:
**26.03.86 Patentblatt 86/13**

(45) Bekanntmachung des Hinweises auf die Patenterteilung:
**07.12.88 Patentblatt 88/49**

(84) Benannte Vertragsstaaten:
**AT BE DE FR GB IT**

(56) Entgegenhaltungen:
EP-A- 0 011 286
DE-C- 2 910 385

**ULLMANNS ENZYKLOPÄDIE DER TECHNISCHEN
CHEMIE, 4. AUFLAGE, 1981, BAND 6, SEITEN 305-306**

**Die Akte enthält technische Angaben, die nach dem
Eingang der Anmeldung eingereicht wurden und die
nicht in dieser Patentschrift enthalten sind.**

(73) Patentinhaber: **BASF Aktiengesellschaft,
Carl-Bosch-Strasse 38, D-6700 Ludwigshafen (DE)**

(72) Erfinder: **Block, Ulrich, Dr., Ungsteiner Strasse 14,
D-6700 Ludwigshafen (DE)**
Erfinder: **Muff, Wolf, Von-Goethe-Strasse 10,
D-6711 Laumersheim (DE)**
Erfinder: **Nixdorf, Klaus, Rilkestrasse 3,
D-6712 Bobenheim-Roxheim (DE)**
Erfinder: **Wagner, Joachim, Mundenheimer Strasse 158,
D-6700 Ludwigshafen (DE)**

EP 0 175 247 B1

## Beschreibung

Die Erfindung betrifft ein Verfahren zur Erzeugung von Unterdruck in Apparaten zur Reinigung, Aufkonzentrierung, Dehydratisierung und Destillation der vorher gewonnenen Rohprodukte von Phthalsäureanhydrid, Maleinsäure und Maleinsäureanhydrid bei der Herstellung von Phthalsäureanhydrid (PSA) und Maleinsäureanhydrid (MSA) durch katalytische Luftoxidation von Naphthalin oder o-Xylol.

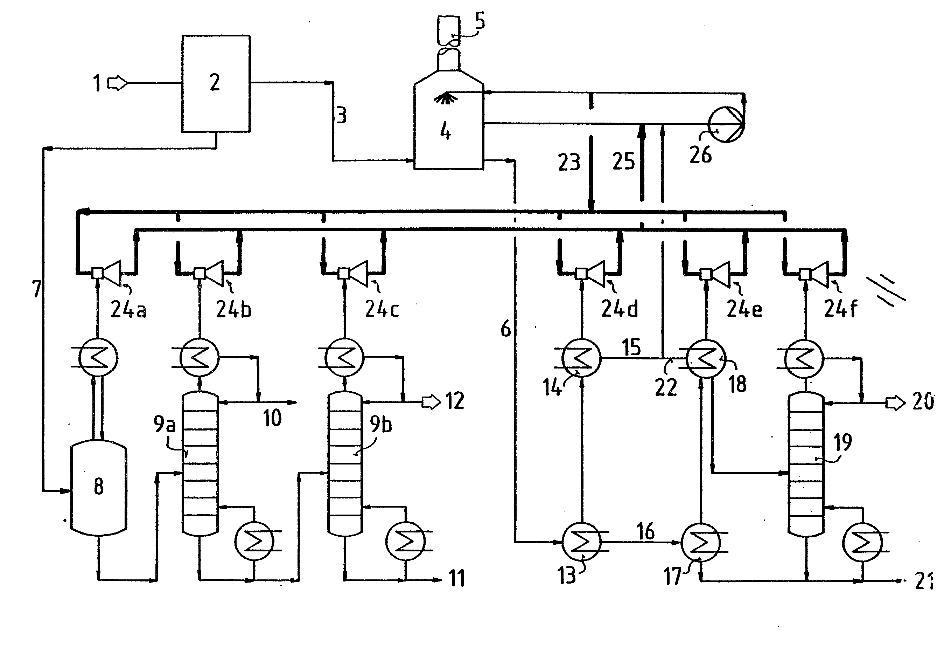

Bei dem Verfahren zur Herstellung von PSA durch katalytische Luftoxidation von Naphthalin oder o-Xylol – wie in dem Nachschlagewerk «Ullmanns Encyklopädie der technischen Chemie», 4. Auflage, Band 18, Seiten 526–532, beispielsweise in Abb. 4, Seite 527, beschrieben – wird aus dem Reaktoraustrittsgas PSA durch Abkühlung in Abscheidern abgetrennt, während MSA aus diesem Gas durch eine nachgeschaltete Abgaswäsche entfernt wird. Aus dem unreinen PSA wird durch thermische Vorbehandlung und anschliessende Destillation reines PSA gewonnen. Die nach der Abgaswäsche anfallende Maleinsäurelösung wird durch Wasserverdampfung aufkonzentriert, die konzentrierte Maleinsäure verdampft und aus dem so erhaltenen Dampf durch Teilkondensation ein noch unsauberes MSA auskondensiert, das anschliessend durch Destillation gereinigt wird.

Die oben beschriebene Herstellung von reinem PSA und reinem MSA erfolgt teilweise bei Unterdruck gegenüber dem atmosphärischen Druck, wobei Absolutdrucke von 1000 bis 100 mbar angewendet werden. Zur Erzeugung dieses Vakuums kommen Wasserringpumpen oder ein- oder zweistufige Dampfstrahlaggregate zur Anwendung.

In der Patentschrift DE 29 10 385 als vorteilhaft beschrieben, Gasstrahlpumpen zur Erzeugung des Vakuums einzusetzen, wobei als Treibmittel Druckluft verwendet wird, und wobei die mit den abgesaugten Gasen beladene Druckluft in die Abgaswaschanlage geführt wird.

Als Nachteil erweist sich hierbei, dass einerseits bei Einsatz von Wasserringpumpen zur Erzeugung der Vakua eine wässrige Lösung anfällt, die einen Teil der abgesaugten Säuren enthält und dabei entsorgt werden muss, und dass andererseits bei Einsatz von Dampfstrahlaggregaten bzw. Gasstrahlpumpen zur Erzeugung der Vakua teure Treibmittel verwendet werden müssen, wobei zudem die dann säurehaltigen Fördermedien entsorgt werden müssen. Zusätzlich erwies sich als Nachteil, dass die Installation und der Betrieb von Wasserringpumpen, Dampfstrahlaggregaten und Gasstrahlpumpen kostenungünstig ist.

Der Erfindung liegt daher die Aufgabe zugrunde, den Aufwand für Installation und Betrieb der Anlagenteile zur Erzeugung von Unterdruck in Apparaturen zur Herstellung von PSA und MSA zu verringern. Ausserdem soll die Entsorgung des Fördermediums zur Erzeugung der Vakua vereinfacht werden.

Diese Aufgabe wird erfindungsgemäss dadurch gelöst, dass zur Erzeugung des Unterdrucks in den Apparaten Flüssigkeitsstrahler verwendet werden, wobei als Fördermedium für die Flüssigkeitsstrahler Waschflüssigkeit aus der Abgaswaschanlage nach den Phthalsäureanhydrid-Abscheidern entnommen wird, die danach wieder in die Abgaswaschanlage zurückgeführt wird.

Weitere Merkmale des erfindungsgemässen Verfahrens sind Gegenstand der Unteransprüche.

Grundgedanke der Erfindung ist, zur Erzeugung von Unterdruck in Apparaten zur Herstellung von PSA und MSA Flüssigkeitsstrahler einzusetzen, wobei als Fördermedium Waschflüssigkeit aus der Abgaswaschanlage verwendet wird, sich diese Waschflüssigkeit während der Vakuumerzeugung mit abgesaugten Anhydriden und Säuren anreichert und anschliessend wieder in die Abgaswaschanlage zurückgeführt wird.

Dadurch wird vermieden, dass die bei den herkömmlichen Verfahren verwendeten Fördermedien, wie beispielsweise Wasser, Wasserdampf oder Inertgase – nach der Erzeugung des Vakuums angereichert mit Anhydriden und Säuren – entsorgt werden müssen, d.h. die dafür bisher notwendigen Anlagenteile können entfallen. Des weiteren erweist sich als vorteilhaft, dass der Energieverbrauch zum Betrieb der Flüssigkeitsstrahler deutlich geringer ist, als bei den bisher verwendeten Anlagenteilen, wie beispielsweise Wasserringpumpen, Dampfstrahlaggregaten oder Gasstrahlpumpen.

Ein Ausführungsbeispiel des erfindungsgemässen Verfahrens ist in der Zeichnung dargestellt und wird im folgenden näher beschrieben. Die Zeichnung zeigt ein vereinfachtes Verfahrensschema.

Gemäss dem Verfahrensschema wird das aus dem Reaktor austretende und anschliessend auf ca. 180 °C gekühlte Reaktionsgas 1 in den Abscheider 2 geleitet, in dem ungereinigtes PSA fest abgeschieden wird. Der im Abscheider auf ca. 50 °C abgekühlte Gasstrom 3 wird zur Abgaswaschanlage 4 geführt, wo der Gasstrom durch eine Wasserwäsche gereinigt wird und über den Kamin 5 in die Umgebung abgegeben wird, während die bei der Wäsche anfallende, wässrige Maleinsäure aus der Abgaswaschanlage als Strom 6 abgezogen wird. Das ungereinigte PSA wird im Abscheider 2 abgeschmolzen und als Strom 7 in die Vorbehandlungsapparatur 8 und die Destillationskolonnen 9a und 9b geführt und reindestilliert, wobei als Strom 10 leichtsiedende Verunreinigungen, als Strom 11 schwersiedende Rückstände und als Strom 12 reines PSA abgezogen wird. Die wässrige Maleinsäure 6 wird im Verdampfer 13 unter Vakuum aufkonzentriert. Das verdampfende Wasser wird im Kondensator 14 kondensiert und als Strom 15 abgezogen und der Abgaswaschanlage zugeführt. Die aufkonzentrierte Maleinsäure gelangt als Strom 16 in den Verdampfer 17, in dem durch Wärmezufuhr unter Vakuum die Maleinsäure in MSA und Wasser aufgespalten wird. MSA und Wasser werden dem Kondensator 18 zugeführt, in dem unter Vakuum ungereinigtes MSA auskondensiert wird, das anschliessend in der Destillationskolonne 19 unter Vakuum in reines MSA als Kopfprodukt 20 und schwersiedende Rückstände 21 getrennt wird. Der aus dem Kondensator 18 abgezogene Strom wird einer weiteren, bei tieferer Temperatur arbeitenden Kondensationsanlage – zeichnerisch nicht dargestellt – zugeführt, kondensiert und anschliessend als Strom 22 der Abgaswaschanlage zugeführt.

Zur Erzeugung des Unterdrucks gegen die Atmosphäre wird aus der Abgaswaschanlage ein Strom 23 abgezogen, der als Fördermedium durch die Flüssigkeitsstrahler 24a, b, c, d, e, f geführt wird, die mit den entsprechenden, unter Vakuum stehenden, Anlagenteile verbunden sind. Angereichert mit Säuren wird der Strom 25 mittels der Pumpe 26 zur Abgaswaschanlage zurückgeführt.

## Patentansprüche

1. Verfahren zur Erzeugung von Unterdruck in Apparaten zur Reinigung, Aufkonzentrierung, Dehydratisierung und Destillation der vorher gewonnenen Rohprodukte von Phthalsäureanhydrid, Maleinsäure und Maleinsäureanhydrid bei der Herstellung von Phthalsäureanhydrid und Maleinsäureanhydrid durch katalytische Luftoxidation von Naphthalin oder o-Xylol, dadurch gekennzeichnet, dass zur Erzeugung des Unterdrucks in den Apparaten Flüssigkeitsstrahler verwendet werden, wobei als Fördermedium für die Flüssigkeitsstrahler Waschflüssigkeit aus der Abgaswaschanlage nach den Phthalsäureanhydrid-Abscheidern entnommen wird, die danach wieder in die Abgaswaschanlage zurückgeführt wird.

2. Verfahren nach Anspruch 1, dadurch gekennzeichnet, dass zwischen dem unter Vakuum stehenden Apparat und dem Flüssigkeitsstrahler ein Dampfstrahlaggregat derart geschaltet wird, dass das Dampfstrahlaggregat aus dem Apparat absaugt und in den Flüssigkeitsstrahler fördert.

3. Verfahren nach den Ansprüchen 1 und 2, dadurch gekennzeichnet, dass zwischen Dampfstrahlaggregat und Flüssigkeitsstrahler ein Kondensator geschaltet wird, wodurch ein weitgehendes Verflüssigen der von dem Dampfstrahlaggregat abgegebenen Dämpfe erreicht wird.

4. Verfahren nach den Ansprüchen 1 bis 3, dadurch gekennzeichnet, dass zwischen dem unter Vakuum stehenden Apparat und dem Flüssigkeitsstrahler mehrere Dampfstrahlaggregate mit jeweils nachgeschaltetem Kondensator angeordnet werden.

## Revendications

1. Procédé d'obtention d'une dépression dans des appareils, lors de la préparation d'anhydrique phtalique et/ou maléique par oxydation catalytique à l'air de naphtalène ou o-Xylène, caractérisé par le fait que, pour l'obtention de la dépression dans les appareils, on utilise des injecteurs de liquide, du liquide de lavage étant prélevé des installations de lavage des gaz résiduaire comme fluide de propulsion pour les injecteurs de liquide, et étant ensuite renvoyé dans les installations de lavage des gaz résiduaires.

2. Procédé selon la revendication 1, caractérisé par le fait qu'entre l'appareil se trouvant sous vide et l'injecteur de liquide est interposé un groupe à jet de vapeur de manière qu'il aspire de l'appareil et refoule dans l'injecteur de liquide.

3. Procédé selon les revendications 1 et 2, caractérisé par le fait qu'entre le groupe à jet de vapeur et l'injecteur de liquide est interposé un condenseur, grâce à quoi on obtient une large condensation de la vapeur dégagée par le groupe à jet de vapeur.

4. Procédé selon les revendications 1 à 3, caractérisé par le fait qu'entre l'appareil se trouvant sous vide et l'injecteur de liquide sont interposés plusieurs groupes à jet de vapeur, à la suite de chacun desquels est placé un condenseur.

## Claims

1. A method for generating reduced pressure in apparatuses in the preparation of phthalic anhydride and/or maleic anhydride by catalytic oxidation of naphthalene or o-xylene with air, wherein the reduced pressure is generated in apparatuses using liquid jet pumps, wash liquid being removed from the exit gas washer as a pump fluid for the liquid jet pumps and subsequently being recycled to the exit gas washer.

2. A method as claimed in claim 1, wherein a steamjet pump is connected between the apparatus under reduced pressure and the liquid jet pump in such a way that the steam-jet pump extracts from the apparatus and delivers into the liquid-jet pump.

3. A method as claimed in claim 1 or 2, wherein a condenser is connected between the steam-jet pump and the liquid-jet pump, by means of which condenser substantial liquefaction of the vapors released by the steam-jet pump is achieved.

4. A method as claimed in claim 1 or 2 or 3, wherein a plurality of steam-jet pumps, each having a downstream condenser, are arranged between the apparatus under reduced pressure and the liquid-jet pump.